(19) Europäisches Patentamt
European Patent Office
Office européen des brevets

(11) **EP 2 910 954 B1**

(12) **EUROPEAN PATENT SPECIFICATION**

(45) Date of publication and mention
of the grant of the patent:
**13.04.2016 Bulletin 2016/15**

(51) Int Cl.:
***G01P 15/18*** *(2006.01)*    ***A61B 5/11*** *(2006.01)*
***G01C 22/00*** *(2006.01)*

(21) Application number: **15155635.4**

(22) Date of filing: **18.02.2015**

(54) **Method for counting steps and electronic apparatus using the same**

Verfahren zum Zählen von Schritten und elektronische Vorrichtung dafür

Procédé de comptage de pas et appareil électronique utilisant celui-ci

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.02.2014 TW 103106314**

(43) Date of publication of application:
**26.08.2015 Bulletin 2015/35**

(73) Proprietor: **Acer Incorporated**
**New Taipei City 221 (TW)**

(72) Inventors:
• **Chen, Yen-Wen**
**New Taipei City 221 (TW)**
• **Lin, Rung-Lung**
**New Taipei City 221 (TW)**

(74) Representative: **2K Patentanwälte Blasberg Kewitz & Reichel**
**Partnerschaft mbB**
**Schumannstrasse 27**
**60325 Frankfurt am Main (DE)**

(56) References cited:
**EP-A1- 1 994 883**    **US-A1- 2006 174 685**
**US-A1- 2012 136 573**    **US-A1- 2012 295 552**

**Description**

BACKGROUND

Technical Field

The invention relates to a method for counting steps and an electronic apparatus using the same. Particularly, the invention relates to a method for counting steps according to signals detected by a microelectromechanical system (MEMS) sensor and an electronic apparatus using the same.

Related Art

A step counter is a device capable of counting steps of a user when the user walks or runs. Conventionally, the step counter has many methods for counting steps. Among these methods, a most direct one is to place an object having a weight in the step counter, and measure a bounce rate of the object according to, for example, an electrical method or a mechanical method.

However, the step counter is generally designed in a larger size in order to accommodate the above object and measuring components. If the step counter is deigned in a smaller size, the method for counting steps adopted by the step counter has to be modified to a method for counting the steps according to signals detected by a microelectromechanical system (MEMS) sensor. The MEMS sensor is, for example, a detecting device such as an accelerometer, a magnetometer, a gyroscope, etc.

EP-A1-1 994 883 describes a body movement detector including a step counter with a three-axis accelerometer. When counting the steps gravity components are considered with the three-axis acceleration values. It is further determined if the user is in a walking or running status.

SUMMARY

The invention is directed to a method for counting steps and an electronic apparatus using the same, by which a step counting result is accurately generated according to signals detected by a microelectromechanical system (MEMS) sensor.

The invention provides a method for counting steps, which is adapted to an electronic apparatus, and the method includes following steps. An orientation and a plurality of first three-axis acceleration values of the electronic apparatus are obtained. A specific ratio of an acceleration of gravity is removed from each of the first three-axis acceleration values according to the orientation to generate a plurality of second three-axis acceleration values. A plurality of inner product values and a plurality of outer product values are calculated according to the second three-axis acceleration values. It is determined whether a user of the electronic apparatus is in a walking status according to the second three-axis acceleration values. If yes, the inner product values are set as reference values, and if not, the outer product values are set as the reference values. A number of steps corresponding to the second three-axis acceleration values is calculated according to the reference values.

The invention provides an electronic apparatus including a detection unit, a storage unit and a processing unit. The detection unit detects an orientation and a plurality of first three-axis acceleration values of the electronic apparatus. The storage unit stores a plurality of modules. The processing unit is coupled to the detection unit and the storage unit, and accesses the modules to execute the following steps. The orientation and a plurality of first three-axis acceleration values of the electronic apparatus are obtained. A specific ratio of acceleration of gravity is removed from each of the first three-axis acceleration values according to the orientation to generate a plurality of second three-axis acceleration values. A plurality of inner product values and a plurality of outer product values are calculated according to the second three-axis acceleration values. It is determined whether a user of the electronic apparatus is in a walking status according to the second three-axis acceleration values. If yes, the inner product values are set as reference values, and if not, the outer product values are set as the reference values. A number of steps corresponding to the second three-axis acceleration values is calculated according to the reference values.

According to the above descriptions, by calculating the number of walking or running steps according to the inner product values and the outer product values, the method for counting steps of the invention can be used to accurately generate the step counting result.

BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.

FIG. 1 is a functional block diagram of an electronic apparatus according to an embodiment of the invention.

FIG. 2 is a flowchart illustrating a method for counting steps according to an embodiment of the invention.

FIG. 3A is a schematic diagram of a plurality of first three-axis acceleration values according to an embodiment of the invention.

FIG. 3B is a schematic diagram of completely removing the acceleration of gravity from the first three-axis acceleration values according to an embodiment of the invention.

FIG. 3C is a schematic diagram of removing a specific ratio of the acceleration of gravity from the first three-axis acceleration values according to an embodiment of the invention.

FIG. 4 is a schematic diagram of calculating inner product values and outer product values according to second three-axis acceleration values according to an embodiment of the invention.

FIG. 5 is a schematic diagram of calculating inner product values and outer product values according to second three-axis acceleration values according to an embodiment of the invention.

FIG. 6 is a flowchart illustrating details of the embodiment of FIG. 2.

FIG. 7 is a schematic diagram of accumulating a counting value according to slope values according to an embodiment of the invention.

FIG. 8 is a schematic diagram of accumulating a counting value according to slope values according to an embodiment of the invention.

FIG. 9 is a schematic diagram of counting steps according to the first three-axis acceleration values according to an embodiment of the invention.

FIG. 10 is a schematic diagram of counting steps according to the first three-axis acceleration values according to an embodiment of the invention.

## DETAILED DESCRIPTION OF DISCLOSED EMBODIMENTS

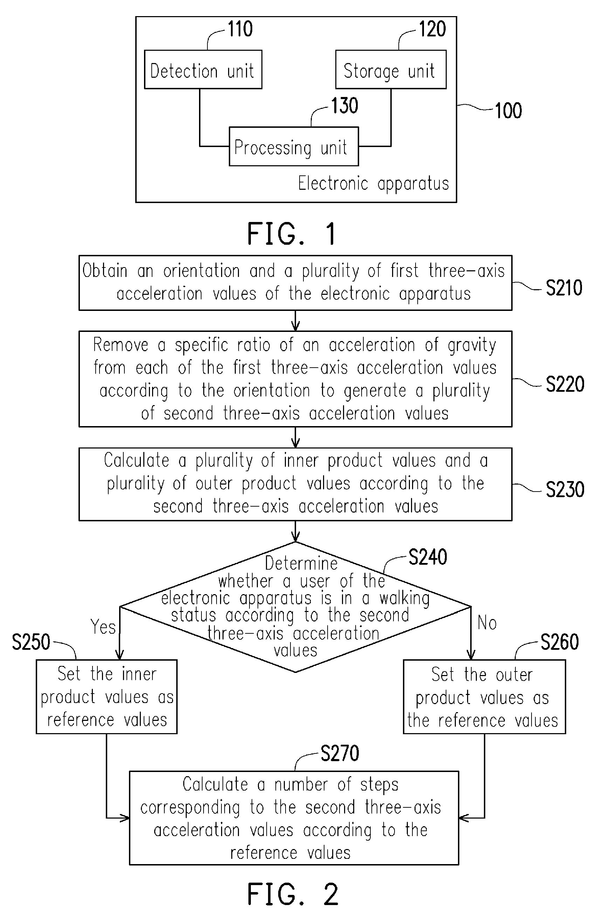

FIG. 1 is a functional block diagram of an electronic apparatus according to an embodiment of the invention. In the present embodiment, the electronic apparatus 100 includes a detection unit 110, a storage unit 120 and a processing unit 130. The electronic apparatus 100 is, for example, a smart phone, a tablet personal computer (PC), a watch, a step counter, a personal digital assistant (PDA), a wearable device, or other similar product.

The detection unit 110 is, for example, an accelerometer, a magnetometer, a gyroscope, etc., or combination thereof, though the invention is not limited thereto. The detection unit 110 can detect an orientation and a plurality of first three-axis acceleration values of the electronic apparatus 100. The orientation is, for example, an azimuth angle of the electronic apparatus 100 in a three-dimensional space. The first three-axis acceleration values are, for example, a plurality of three-axis acceleration sampling values of the electronic apparatus captured by the detection unit 110 at different time points, though the invention is not limited thereto. The first three-axis acceleration values can be respectively represented by an X-axis acceleration component, a Y-axis acceleration component and a Z-axis acceleration component. According to another aspect, when a user carries the electronic apparatus 100 to conduct activities such as walking or running, etc., the detection unit 110 can detect a moving status (i.e. the orientation and the first three-axis acceleration values) of the electronic apparatus 100 generated due to the activity of the user.

The storage unit 120 is, for example, a fixed or movable random access memory (RAM) of any type, a read-only memory (ROM), a flash memory, a hard disk or other similar devices or a combination of the devices, which is not limited by the invention.

The processing unit 130 is coupled to the detection unit 110 and the storage unit 120. The processing unit 130 is, for example, a single chip, a general-purpose processor, a special-purpose processor, a conventional processor, a digital signal processor, a plurality of microprocessors, one or a plurality of microprocessors combined with a digital signal processor core, a controller, a micro controller, an application specific integrated circuit (ASIC), a field programmable gate array (FPGA), any other type of integrated circuit, a state machine, a processor based on an advanced RISC machine (ARM) and similar products.

In the present embodiment, the processing unit 130 may access a plurality of modules stored in the storage unit 120 to execute various steps of the method for counting steps provided by the invention.

FIG. 2 is a flowchart illustrating a method for counting steps according to an embodiment of the invention. The method provided by the present embodiment can be executed by the electronic apparatus 100 of FIG. 1, and detailed steps of the method are described below with reference of various components shown in FIG. 1.

First, in step S210, the processing unit obtains the orientation and a plurality of the first three-axis acceleration values of the electronic apparatus 100 from the detection unit 110. Then, in step S220, the processing unit 130 removes a specific ratio of acceleration of gravity from each of the first three-axis acceleration values according to the orientation to generate a plurality of second three-axis acceleration values. The second three-axis acceleration values can be respectively represented by an X-axis acceleration component, a Y-axis acceleration component and a Z-axis acceleration component. The specific ratio can be 70%, though the invention is not limited thereto.

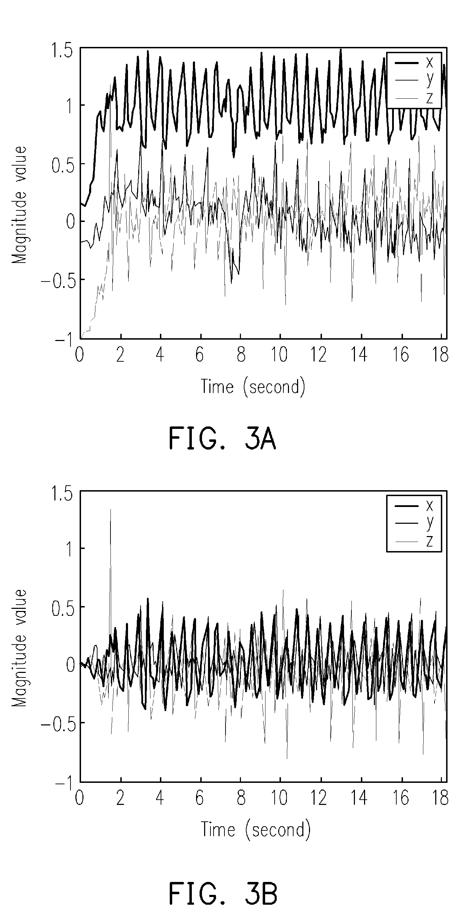

In other words, the processing unit 130 only removes a part of the acceleration of gravity from each of the first three-axis acceleration values, and does not completely remove the acceleration of gravity from each of the first three-axis acceleration values. A reason thereof is that if the acceleration of gravity is completely removed from each of the first three-axis acceleration values, each of the second three-axis acceleration values is rather close to a noise value to cause a wrong step counting result, and details thereof are described with reference of FIG. 3A to FIG. 3C.

FIG. 3A is a schematic diagram of a plurality of first three-axis acceleration values according to an embodiment of the invention. In the present embodiment, a vertical axis represents a magnitude of the first-axis acceleration values, and a unit thereof is, for example, g (i.e. 9.8 metre/second$^2$), and a horizontal axis is a time axis with a unit of, for example, second. A corresponding relationship between the X-axis acceleration component of each of the first three-axis acceleration values and the time axis is shown as a curve x; a corresponding relationship between the Y-axis acceleration component of each of the first three-axis acceleration values and the time axis is shown as a curve y; and a corresponding relationship between the Z-axis acceleration component of each of the first three-axis acceleration values and the time axis is shown as a curve z. According to FIG. 3A, it is known that an average of the curve x is about 1 g, an average of the curve y or the curve z is about 0 g. Therefore, based on different trends of the curves x, y and z, it is known that the curve x is more related to the activity of the user, which may provide more information required for counting the steps.

Referring to FIG. 3B, FIG. 3B is a schematic diagram of completely removing the acceleration of gravity from the first three-axis acceleration values according to an embodiment of the invention. According to FIG. 3B, when the processing unit 130 completely removes the acceleration of gravity from each of the first three-axis acceleration values, the curve x is very close to the curve y and the curve z, which results in a fact that the processing unit 130 generates a wrong step counting result while counting the steps.

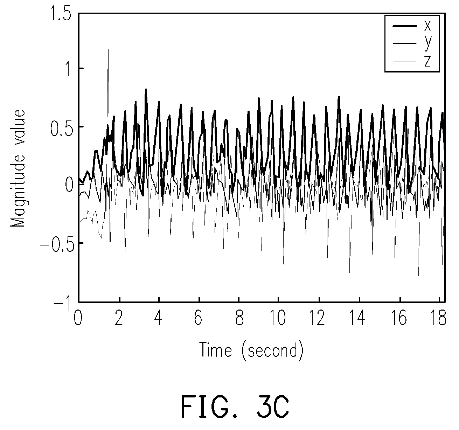

Referring to FIG. 3B, FIG. 3C is a schematic diagram of removing a specific ratio of the acceleration of gravity from the first three-axis acceleration values according to an embodiment of the invention. According to FIG. 3C, when the processing unit 130 only removes the specific ratio (for example, 70%) of the acceleration of gravity from each of the first three-axis acceleration values, the curve x is still separated from the curve y and the curve z, and the processing unit 130 can generate an accurate step counting result while counting the steps.

Referring to FIG. 2 again, in step S230, the processing unit 130 calculates a plurality of inner product values and a plurality of outer product values according to the second three-axis acceleration values. Similar to the first three-axis acceleration values, each of the second three-axis acceleration values can also be regarded as a sampling value corresponding to different time points. Therefore, the processing unit 130 can perform an inner production operation on the second-axis acceleration values corresponding to two continuous time points to calculate the inner product value.

For example, it is assumed that an i$^{th}$ (i is a positive integer) second three-axis acceleration value is represented as $(x_i, y_i, z_i)$, where $x_i$ is the X-axis acceleration component of the i$^{th}$ second three-axis acceleration value, $y_i$ is the Y-axis acceleration component of the i$^{th}$ second three-axis acceleration value, and $z_i$ is the Z-axis acceleration component of the i$^{th}$ second three-axis acceleration value. Moreover, it is assumed that an (i-1)$^{th}$ second three-axis acceleration value is represented as $(x_{i-1}, y_{i-1}, z_{i-1})$, where $x_{i-1}$ is the X-axis acceleration component of the (i-1)$^{th}$ second three-axis acceleration value, $y_{i-1}$ is the Y-axis acceleration component of the (i-1)$^{th}$ second three-axis acceleration value, and $z_{i-1}$ is the Z-axis acceleration component of the (i-1)$^{th}$ second three-axis acceleration value. Based on $(x_i, y_i, z_i)$ and $(x_{i-1}, y_{i-1}, z_{i-1})$, the processing unit 130 can calculate an i$^{th}$ inner product value and an i$^{th}$ outer product value. The i$^{th}$ inner product value is, for example, $\dfrac{\left(x_i x_{i-1} + y_i y_{i-1} + z_i z_{i-1}\right)^2}{\left(x_i^2 + y_i^2 + z_i^2\right)\left(x_{i-1}^2 + y_{i-1}^2 + z_{i-1}^2\right)}$, and the i$^{th}$ outer product value is, for example,

$[(y_i z_i - z_i y_{i-1})^2 + (z_i x_{i-1} - x_i z_{i-1})^2 + (x_i y_{i-1} - y_i x_{i-1})^2]^2$, though the invention is not limited thereto.

Then, in step S240, the processing unit 130 determines whether the user of the electronic apparatus 100 is in a walking status according to the second three-axis acceleration values. In detail, the processing unit 130 can calculate a plurality of magnitude values corresponding to the second three-axis acceleration values. Taking the i$^{th}$ second three-axis acceleration value as an example, the magnitude value thereof is, for example, $\sqrt{\left(x_i^2 + y_i^2 + z_i^2\right)}$, though the invention is not limited thereto. Then, the processing unit 130 calculates an average of the magnitude values, and determines whether the average is higher than a first predetermined threshold value (for example, 0.2). If yes, the processing unit 130 determines that the user is in the walking status, and a step S250 is executed. If not, the processing unit 130 determines that the user is not in the walking status (i.e. the user is probably in a running status), and a step S260 is executed.

In detail, when the user walks while wearing, holding or carrying the electronic apparatus 100, since walking is a gentler activity compared with that of running, the average of the second three-axis acceleration values calculated by the processing unit 130 is smaller. Conversely, when the user runs while carrying the electronic apparatus 100, the processing unit 130, the average of the second three-axis acceleration values calculated by the processing unit 130 is greater. Therefore, the processing unit 130 can determine whether the user is in the walking status by determining whether the average of the second three-axis acceleration values is greater than the first predetermined threshold value.

In the step S250, the processing unit 130 sets the inner product values as reference values. On the other hand, in step S260, the processing unit 130 sets the outer product values as the reference values. After the steps S250 and S260, the processing unit 130 executes a step S270 to calculate a number of steps corresponding to the second three-axis acceleration values according to the reference values.

According to another aspect, when the processing unit 130 obtains each of the second three-axis acceleration values of the electronic apparatus 100, the processing unit 130 simultaneously calculates the inner product value and the outer product value corresponding to each of the second three-axis acceleration values. Then, when the processing unit 130 determines that the user is in the walking status, the processing unit 130 can calculate the number of steps according to each of the inner product values. When the processing unit 130 determines that the user is not in the walking status, the processing unit 130 can calculate the number of steps according to each of the outer product values.

In detail, when the user is in the walking status, the second three-axis acceleration values calculated by the processing unit 130 present a smaller fluctuation, and the second three-axis acceleration values have a smaller noise. In this case, if the processing unit 130 calculates the number of steps according to the outer product values corresponding to the second three-axis acceleration values, since information used for counting the number of steps is probably eliminated when the outer product values are calculated, a wrong number of steps is probably derived. However, if the number of steps is calculated according to the inner product values corresponding to the second three-axis acceleration values, the information used for counting the number of steps can be effectively retained, so as to produce an accurate step counting result.

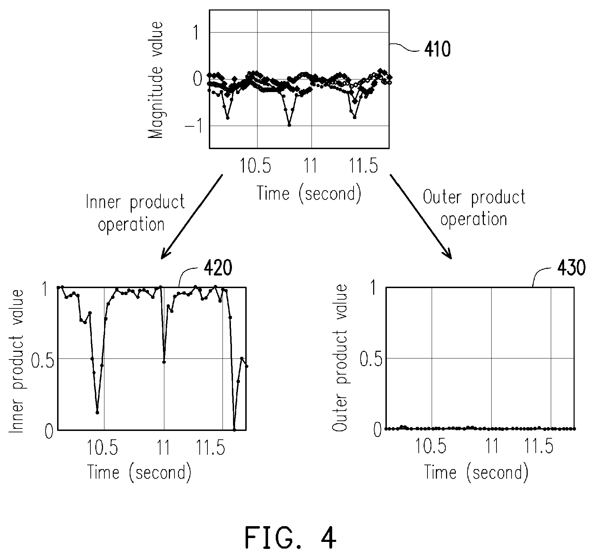

FIG. 4 is a schematic diagram of calculating the inner product values and the outer product values according to the second three-axis acceleration values according to an embodiment of the invention. In the present embodiment, a chart 410 illustrates a corresponding relationship between each of the second three-axis acceleration values and the time axis. A chart 420 illustrates a plurality of inner product values calculated by the processing unit 130 according to each of the second three-axis acceleration values in the chart 410. A chart 430 illustrates a plurality of outer product values calculated by the processing unit 130 according to each of the second three-axis acceleration values in the chart 410.

In detail, a pattern presented by each of the second three-axis acceleration values in the chart 410, for example, corresponds to the walking status of the user. In this case, the chart 420 calculated by the processing unit 130 can still retain a pattern trend similar as that of the chart 410. Referring to the chart 430, since the information used for counting the number of steps is probably eliminated when the outer product values are calculated, a pattern trend of the chart 430 is quite different with that of the chart 410. Therefore, when the processing unit 130 determines that the user is in the walking status, the processing unit 130 calculates the number of steps according to the inner product values in the chart 420, so as to obtain an accurate step counting result.

On the other hand, when the user is in the running status, the second three-axis acceleration values calculated by the processing unit 130 present a larger fluctuation, and the second three-axis acceleration values have a larger noise. In this case, if the processing unit 130 still calculates the number of steps according to the inner product values corresponding to the second three-axis acceleration values, a wrong number of steps is probably derived due to excessive noise. However, if the number of steps is calculated according to the outer product values corresponding to the second three-axis acceleration values, the negative influence on determination of the number of steps caused by the noise can be effectively avoided, so as to produce the accurate step counting result.

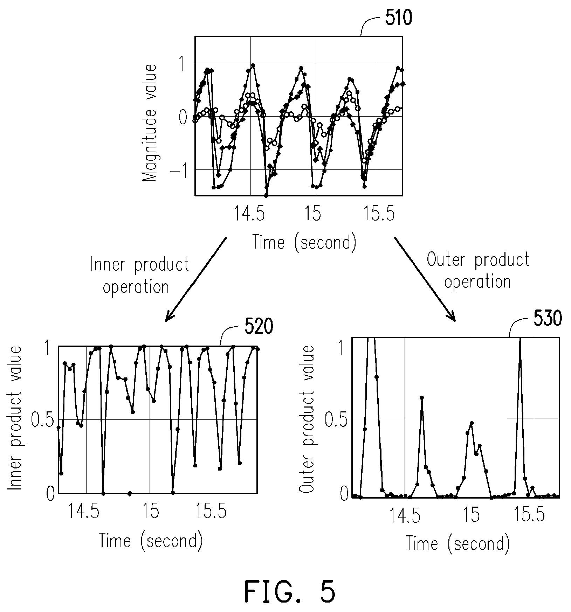

FIG. 5 is a schematic diagram of calculating the inner product values and the outer product values according to the second three-axis acceleration values according to an embodiment of the invention. In the present embodiment, a chart 510 illustrates a corresponding relationship between each of the second three-axis acceleration values and the time axis. A chart 520 illustrates a plurality of inner product values calculated by the processing unit 130 according to each of the second three-axis acceleration values in the chart 510. A chart 530 illustrates a plurality of outer product values calculated by the processing unit 130 according to each of the second three-axis acceleration values in the chart 510.

In detail, a pattern presented by each of the second three-axis acceleration values in the chart 510, for example, corresponds to the running status of the user. In this case, the chart 530 calculated by the processing unit 130 can still retain a pattern trend similar as that of the chart 510. Referring to the chart 520, since the inner product values are influenced by excessive noise when the inner product values are calculated, a pattern trend thereof is quite different with that of the chart 510. Therefore, when the processing unit 130 determines that the user is not in the walking status (i.e. in the running status), the processing unit 130 calculates the number of steps according to the outer product values in the chart 530, so as to obtain an accurate step counting result.

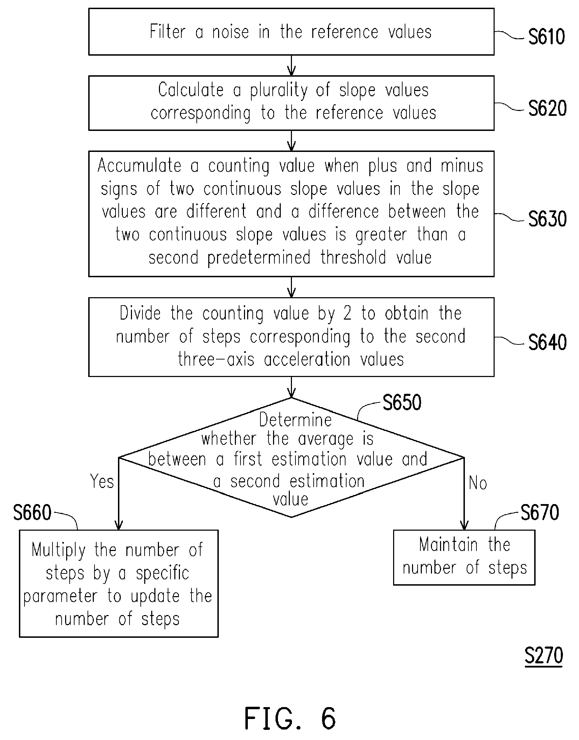

FIG. 6 is a flowchart illustrating details of the embodiment of FIG. 2. In the present embodiment implementation details of the step S270 of FIG. 2 are introduced, though the invention is not limited thereto.

In step S610, the processing unit 130 filters noises in the reference values. For example, the processing unit 130 performs a moving average (MA) operation to each of the reference values to calculate a moving average of each of the reference values. In an embodiment, the MA operation is, for example, a weighted moving average (WMA) operation. Taking the WMA operation of 10 reference values as an example, the moving average corresponding to an $i^{th}$ reference value can be represented as:

$$d1_i = WMA_{10}(d_i) = \frac{10d_i + 9d_{i-1} + \cdots + d_{i-9}}{55}$$

Where $d1_i$ is the moving average corresponding to the $i^{th}$ reference value, $d_i$ is the $i^{th}$ reference value. Those skilled in the art should understand that the MA operation method of the present embodiment is only an example, and the invention is not limited thereto.

Then, in step S620, the processing unit 130 calculates a plurality of slope values corresponding to the reference values. In an embodiment, the processing unit 130 differentiates each of the reference values to obtain the corresponding slope value. Therefore, the slope value corresponding to the $i^{th}$ reference value can be represented as:

$$d2_i = Diff(d1_i) = \frac{1}{8}\left[2d1_i + d1_{i-1} - d1_{i-3} - 2d1_{i-4}\right]$$

Where, $d2_i$ is the slope value corresponding to the $i^{th}$ reference value. Those skilled in the art should understand that the slope value calculation method of the present embodiment is only an example, and the invention is not limited thereto.

Then, in step S630, when plus and minus signs of two continuous slope values are different and a difference between the two continuous slope values is greater than a second predetermined threshold value (for example, 0.2), a counting value is accumulated, where the two continuous slope values are, for example, $d2_i$ and $d_{2i-1}$ (where i is any positive integer).

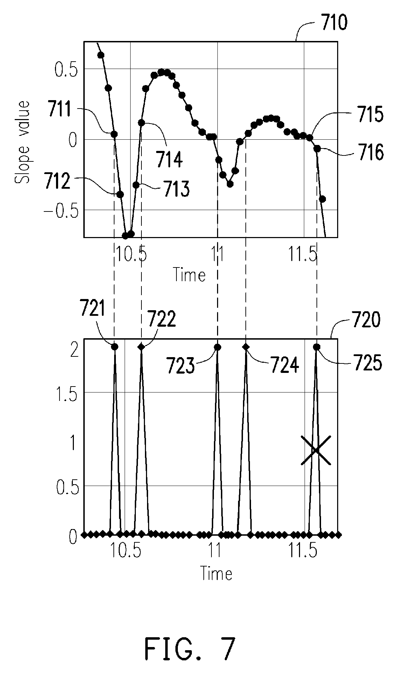

Referring to FIG. 7, FIG. 7 is a schematic diagram of accumulating the counting value according to the slope values according to an embodiment of the invention. In the present embodiment, a chart 710 illustrates a corresponding relationship between each of the slope values and the time axis. Each of peak values 721-725 illustrated in a chart 720 corresponds to a time point when the plus and minus signs of two continuous slope values are different. Taking the peak value 721 as an example, the peak value 721 corresponds to a time point when the slope value is 0 (i.e. a time point when the slope value is changed from a positive value to a negative value). Taking the peak value 722 as an example, the peak value 722 corresponds to a time point when the slope value is 0 (i.e. a time point when the slope value is changed from a negative value to a positive value). Taking the peak value 723 as an example, the peak value 723 corresponds to a time point when the slope value is 0 (i.e. a time point when the slope value is changed from a positive value to a negative value). Taking the peak value 724 as an example, the peak value 724 corresponds to a time point when the slope value is 0 (i.e. a time point when the slope value is changed from a negative value to a positive value). Taking the peak value 725 as an example, the peak value 725 corresponds to a time point when the slope value is 0 (i.e. a time point when the slope value is changed from a positive value to a negative value).

Referring to the chart 710, since the plus and minus signs of the two continuous slope values 711 and 712 are different and a difference between the two continuous slope values 711 and 712 is greater than the second predetermined threshold value (for example, 0.2), the processing unit 130 accumulates the counting value. Then, since the plus and minus signs of the two continuous slope values 713 and 714 are different and a difference between the two continuous slope values 713 and 714 is greater than the second predetermined threshold value (for example, 0.2), the processing unit 130 again accumulates the counting value.

However, although the plus and minus signs of the two continuous slope values 715 and 716 are different, since a difference between the two continuous slope values 715 and 716 is not greater than the second predetermined threshold value (for example, 0.2), the processing unit 130 does not accumulates the counting value. In this way, influence of the step counting result due to slight disturbance can be avoided.

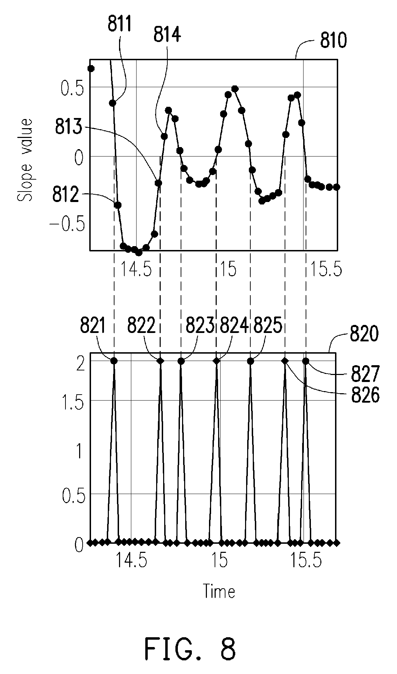

Referring to FIG. 8, FIG. 8 is a schematic diagram of accumulating the counting value according to the slope values according to an embodiment of the invention. In the present embodiment, a chart 810 illustrates a corresponding relationship between each of the slope values and the time axis. Each of peak values 821-827 illustrated in a chart 820 corresponds to a time point when the plus and minus signs of two continuous slope values are different.

Referring to the chart 810, since the plus and minus signs of the two continuous slope values 811 and 812 are different and a difference between the two continuous slope values 811 and 812 is greater than the second predetermined threshold value (for example, 0.2), the processing unit 130 accumulates the counting value. Then, since the plus and minus signs of the two continuous slope values 813 and 814 are different and a difference between the two continuous slope values 813 and 814 is greater than the second predetermined threshold value (for example, 0.2), the processing unit 130 again accumulates the counting value.

Referring to FIG. 6 again, it should be noticed that the two peak values (for example, the peak values 721 and 722) only correspond to one step, and in the step S640, the processing unit 130 can divide the counting value by 2 to obtain the number of steps corresponding to the second three-axis acceleration values.

Moreover, in other embodiments, when the user is changed from the walking status to the running status, or is changed from the running status to the walking status, the processing unit 130 can further execute steps S650-S670 to accurately calculate the corresponding number of steps.

In the step S650, the processing unit 130 determines whether the average is between a first estimation value (for example, 0.2) and a second estimation value (for example, 0.5). If yes, the step S660 is executed, and if not, the step S670 is executed.

In the step S660, the processing unit 130 can multiply the number of steps by a specific parameter to update the number of steps. The specific parameter is, for example, represented as:

$$\frac{\alpha_{motion} - \alpha_{walk}}{\alpha_{motion} - \alpha_{walk} + \varepsilon}$$

Where, $a_{walk}$ is the first estimation value, $a_{motion}$ is the second estimation value, $\varepsilon$ is an error rate estimation value (for example, 0.3). In detail, when the average is between the first estimation value and the second estimation value, it represents that the user is changing from the walking status to the running status, or changing from the running status to the walking status, and the processing unit 130 can multiply the number of steps by the specific parameter to correct the number of steps. In this way, the electronic apparatus 100 can still produce the correct step counting result when the user is changing the motion status.

On the other hand, in the step S670, the processing unit 130 can maintain the number of steps. In detail, when the average is not between the first estimation value and the second estimation value, it represent that the user is walking or running. Therefore, the processing unit 130 does not multiply the number of steps by the specific parameter to correct the number of steps.

By respectively calculating the numbers of walking and running steps according to the inner product values and the outer product values, the method for counting steps of the invention can correctly generate the step counting result. Moreover, by adaptively multiplying the number of steps by the specific parameter, according to the method of the invention, the electronic apparatus 100 can still provide the correct step counting result when the user is changing the motion status.

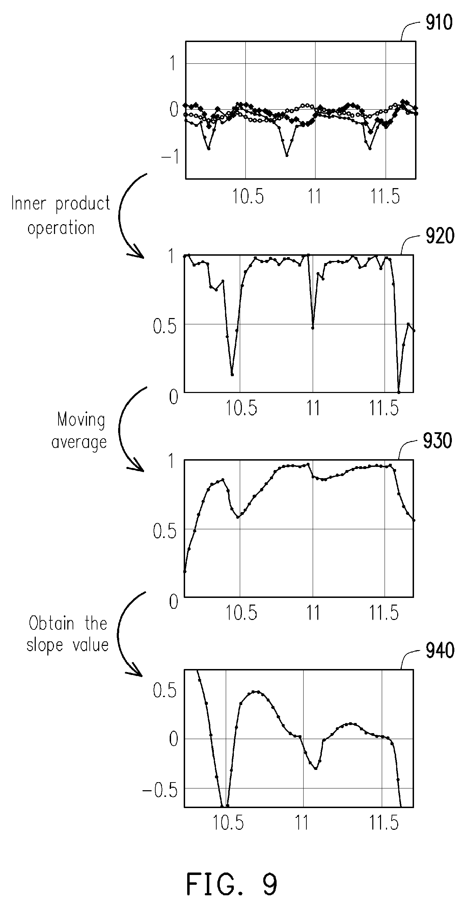

FIG. 9 is a schematic diagram of counting steps according to the first three-axis acceleration values according to an embodiment of the invention. In the present embodiment, a chart 910 illustrates a corresponding relationship between each of the first three-axis acceleration values and the time axis. A chart 920 illustrates the inner product values corresponding to each of the first three-axis acceleration values in the chart 910. A chart 930 illustrates the moving averages corresponding to each of the inner product values in the chart 920. A chart 940 illustrates the slop values corresponding to each of the moving averages in the chart 930.

Since a pattern of the chart 910 can be regarded as corresponding to the walking status, the processing unit 130 can adopt the inner product values of the chart 920 to implement follow-up operations, so as to generate the correct step counting result.

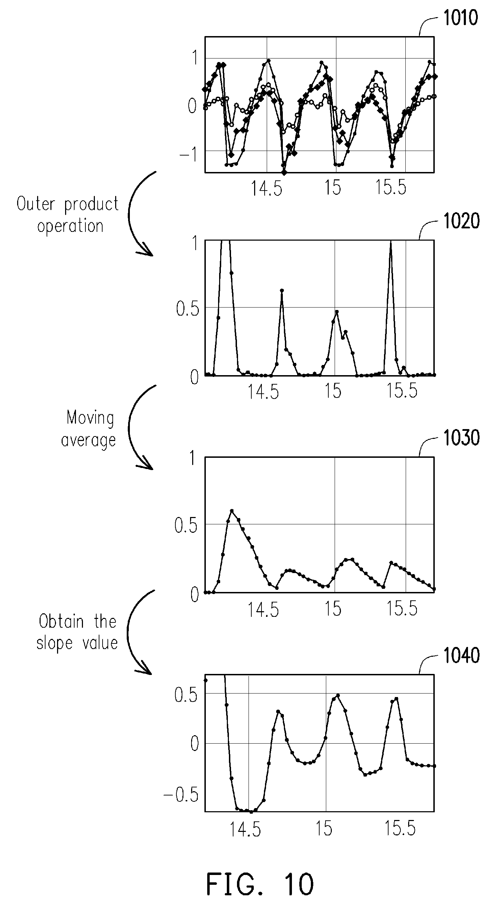

FIG. 10 is a schematic diagram of counting steps according to the first three-axis acceleration values according to an embodiment of the invention. In the present embodiment, a chart 1010 illustrates a corresponding relationship between each of the first three-axis acceleration values and the time axis. A chart 1020 illustrates the outer product values corresponding to each of the first three-axis acceleration values in the chart 1010. A chart 1030 illustrates the moving averages corresponding to each of the outer product values in the chart 1020. A chart 1040 illustrates the slop values corresponding to each of the moving averages in the chart 1030.

Since a pattern of the chart 1010 can be regarded as corresponding to the running status, the processing unit 130 can adopt the outer product values of the chart 1020 to implement follow-up operations, so as to generate the correct step counting result.

In summary, by calculating the number of walking or running steps according to the inner product values and the outer product values, the method for counting steps of the invention can be used to accurately generate the step counting result. Moreover, by adaptively multiplying the number of steps by the specific parameter, according to the method provided by the invention, the electronic apparatus can still provide the correct step counting result when the user is changing the motion status.

**Claims**

1. A method for counting steps, adapted to an electronic apparatus (100), the method for counting steps comprising:

   obtaining (S210) an orientation and a plurality of first three-axis acceleration values of the electronic apparatus (100);
   removing (S220) a specific ratio of an acceleration of gravity from each of the first three-axis acceleration values according to the orientation to generate a plurality of second three-axis acceleration values;
   calculating (S230) a plurality of inner product values and a plurality of outer product values according to the second three-axis acceleration values;
   determining (S240) whether a user of the electronic apparatus (100) is in a walking status according to the second three-axis acceleration values;
   if yes, setting (S250) the inner product values as reference values;
   if not, setting (S260) the outer product values as the reference values; and
   calculating (S270) a number of steps corresponding to the second three-axis acceleration values according to the reference values.

2. The method for counting steps as claimed in claim 1, wherein the step of determining whether the user of the electronic apparatus (100) is in the walking status according to the second three-axis acceleration values comprises:

   calculating a plurality of magnitude values corresponding to the second three-axis acceleration values;
   calculating an average of the magnitude values; and
   determining whether the average is higher than a first predetermined threshold;

      if yes, determining that the user is in the walking status; and
      if not, determining that the user is not in the walking status.

3. The method for counting steps as claimed in claim 1or 2, wherein the step of calculating the number of steps corresponding to the second three-axis acceleration values according to the reference values comprises:

   filtering (S610) a noise in the reference values;
   calculating (S620) a plurality of slope values corresponding to the reference values;
   accumulating (S630) a counting value when plus and minus signs of two continuous slope values in the slope values are different and a difference between the two continuous slope values is greater than a second predetermined threshold value; and
   dividing (S640) the counting value by 2 to obtain the number of steps corresponding to the second three-axis acceleration values.

4. The method for counting steps as claimed in any of the claims 1 to 3, wherein after the step of dividing the counting value by 2 to obtain the number of steps corresponding to the second three-axis acceleration values, the method for counting steps further comprises:

   determining (S650) whether the average is between a first estimation value and a second estimation value;

      if yes, multiplying (S660) the number of steps by a specific parameter to update the number of steps; and
      if not, maintaining (S670) the number of steps.

5. The method for counting steps as claimed in any of the claims 1 to 4, wherein the specific parameter is:

$$\frac{\alpha_{motion} - \alpha_{walk}}{\alpha_{motion} - \alpha_{walk} + \varepsilon}$$

wherein $a_{walk}$ is the first estimation value, $a_{motion}$ is the second estimation value, $\varepsilon$ is an error rate estimation value.

6. An electronic apparatus (100), comprising:

a detection unit (110), detecting an orientation and a plurality of first three-axis acceleration values of the electronic apparatus (100);
a storage unit (120), storing a plurality of modules; and
a processing unit (130), coupled to the detection unit (110) and the storage unit, and accessing the modules to execute following steps:

obtaining (S210) an orientation and a plurality of first three-axis acceleration values of the electronic apparatus (100);
removing (S220) a specific ratio of an acceleration of gravity from each of the first three-axis acceleration values according to the orientation to generate a plurality of second three-axis acceleration values;
calculating (S230) a plurality of inner product values and a plurality of outer product values according to the second three-axis acceleration values;
determining (S240) whether a user of the electronic apparatus (100) is in a walking status according to the second three-axis acceleration values;

if yes, setting (S250) the inner product values as reference values;
if not, setting (S260) the outer product values as the reference values; and
calculating (S270) a number of steps corresponding to the second three-axis acceleration values according to the reference values.

7. The electronic apparatus (100) as claimed in claim 6, wherein the processing unit (130) is configured to:

calculate a plurality of magnitude values corresponding to the second three-axis acceleration values;
calculate an average of the magnitude values; and
determine whether the average is higher than a first predetermined threshold;

if yes, determine that the user is in the walking status; and
if not, determine that the user is not in the walking status.

8. The electronic apparatus (100) as claimed in claim 6 or 7, wherein the processing unit (130) is configured to:

filter (S610) a noise in the reference values;
calculate (S620) a plurality of slope values corresponding to the reference values;
accumulate (S630) a counting value when plus and minus signs of two continuous slope values in the slope values are different and a difference between the two continuous slope values is greater than a second predetermined threshold value; and
divide (S640) the counting value by 2 to obtain the number of steps corresponding to the second three-axis acceleration values.

9. The electronic apparatus (100) as claimed in claim 8, wherein the processing unit (130) is configured to:

determine (S650) whether the average is between a first estimation value and a second estimation value;
if yes, multiply (S660) the number of steps by a specific parameter to update the number of steps; and
if not, maintain (S670) the number of steps.

10. The electronic apparatus (100) as claimed in claim 9, wherein the specific parameter is:

$$\frac{\alpha_{motion} - \alpha_{walk}}{\alpha_{motion} - \alpha_{walk} + \varepsilon}$$

wherein $a_{walk}$ is the first estimation value, $a_{motion}$ is the second estimation value, $\varepsilon$ is an error rate estimation value.

**Patentansprüche**

**1.** Ein Verfahren zum Zählen von Schritten angepasst für eine elektronische Vorrichtung (100), das Verfahren zum Zählen von Schritten umfasst:

Erlangen (S210) einer Ausrichtung und einer Vielzahl von ersten Drei-Achsen-Beschleunigungswerten der elektronischen Vorrichtung (100);
Entfernen (S220) eines bestimmten Verhältnisses einer Beschleunigung der Gravitation von jedem der ersten Drei-Achsen-Beschleunigungswerten entsprechend der Ausrichtung, um eine Vielzahl zweiter Drei-Achsen-Beschleunigungswerte zu erzeugen;
Berechnung (S230) einer Vielzahl von inneren Produktwerten und einer Vielzahl von äußeren Produktwerten gemäß den zweiten Drei-Achsen-Beschleunigungswerten;
Bestimmung (S240), ob ein Benutzer der elektronischen Vorrichtung (100) in einem gehenden Zustand gemäß den zweiten Drei-Achsen-Beschleunigungswerten ist;
falls ja, Einstellung (S250) der inneren Produktwerte als Referenzwerte;
falls nicht, Einstellung (S260) der äußeren Produktwerte als Referenzwerte; und
Berechnung (S270) einer Anzahl von Schritten korrespondierend zu den zweiten Drei-Achsen-Beschleunigungswerten entsprechend den Referenzwerten.

**2.** Das Verfahren zum Zählen von Schritten nach Anspruch 1, wobei der Schritt zur Bestimmung, ob der Benutzer der elektronischen Vorrichtung (100) in einem gehenden Zustand, gemäß den zweiten Drei-Achsen-Beschleunigungswerten, ist, umfasst:

Berechnung einer Vielzahl von Magnitude-Werten entsprechend den zweiten Drei-Achsen-Beschleunigungswerten;
Berechnung eines Mittelwertes der Magnitude-Werte; und
Bestimmen, ob der Durchschnittswert höher als ein erster vorbestimmter Schwellenwert ist;
falls ja, Bestimmung, dass der Benutzer im gehenden Zustand ist; und
falls nicht, Bestimmung, dass der Benutzer nicht im gehenden Zustand ist.

**3.** Das Verfahren zum Zählen von Schritten nach Anspruch 1 oder 2, wobei der Schritt der Berechnung der Anzahl von Schritten entsprechend dem zweiten Drei-Achsen-Beschleunigungswertes entsprechend den Referenzwerten umfasst:

Filterung (S610) eines Geräuschs in den Referenzwerten;
Berechnung (S620) einer Vielzahl von Steigungswerten entsprechend der Referenzwerte;
Akkumulierung (S630) eines Zählwertes wenn Plus- und Minuszeichen von zwei kontinuierlichen Steigungswerte innerhalb der Steigungswerte unterschiedlich sind und ein Unterschied zwischen den zwei kontinuierlichen Steigungswerten größer als ein zweiter vorbestimmter Schwellenwert ist; und
Teilen (S640) der Zählwerte durch 2, um die Anzahl der Schritte entsprechend den zweiten Drei-Achsen-Beschleunigungswerten zu erhalten.

**4.** Das Verfahren zum Zählen von Schritten nach einem der Ansprüche 1 bis 3, wobei nach dem Schritt des Teilens des Zählwertes durch 2, um die Anzahl von Schritten entsprechend den zweiten Drei-Achsen-Beschleunigungswerten zu erhalten, das Verfahren zum Zählen von Schritten umfasst:

Bestimmen (S650), ob der Durchschnitt zwischen einem ersten Schätzwert und einen zweiten Schätzwert liegt;
falls ja, Multiplikation (S660) der Anzahl von Schritten, mit einen bestimmten Parameter, um die Anzahl der Schritte zu aktualisieren; und
falls nicht, Beibehaltung (S670) der Anzahl von Schritten.

**5.** Das Verfahren zum Zählen von Schritten nach einem der Ansprüche 1 bis 4, wobei der spezifische Parameter ist:

$$\frac{\alpha_{motion} - \alpha_{walk}}{\alpha_{motion} - \alpha_{walk} + \varepsilon}$$

wobei $a_{walk}$ der erste Schätzwert ist, $a_{motion}$ ist der zweite Schätzwert, $\varepsilon$ ist ein Fehlerrate-Schätzwert.

6. Eine elektronische Vorrichtung (100), umfassend:

eine Erfassungseinheit (110), die eine Ausrichtung und einer Vielzahl von ersten Drei-Achsen-Beschieunigungswerten der elektronischen Vorrichtung (100) erfasst;
eine Speichereinheit (120), die eine Vielzahl von Modulen erfasst; und
eine Verarbeitungseinheit (130), die mit der Erfassungseinheit (110) und der Speichereinheit verbunden ist, und auf die Module zugreift, um folgende Schritte auszuführen:

Erhalten (S210) einer Ausrichtung und einer Vielzahl von ersten Drei-Achsen-Beschleunigungswerten der elektronischen Vorrichtung (100);
Entfernen (S220) eines spezifischen Verhältnisses einer Beschleunigung einer Gravitation von jeder der ersten Drei-Achsen-Beschleunigungswerten entsprechend der Ausrichtung, um eine Vielzahl zweiter dreiachsiger-Beschleunigungswerte zu erzeugen;
Berechnen (S230) einer Vielzahl von inneren Produktwerten und einer Vielzahl von äußeren Produktwerten entsprechend den zweiten Drei-Achsen-Beschleunigungswerten;
Bestimmen (S240), ob ein Benutzer der elektronischen Vorrichtung (100) in einem gehenden Zustand entsprechend den zweiten Drei-Achsen-Beschleunigungswerten ist;
falls ja, Einstellen (S250) der inneren Produktwerte als Referenzwerte;
falls nicht, Einstellen (S260) der äußeren Produktwerte als Referenzwerte; und
Berechnen (S270) einer Anzahl von Schritten entsprechend den zweiten Drei-Achsen-Beschleunigungswerten gemäß den Referenzwerten.

7. Die elektronische Vorrichtung (100) nach Anspruch 6, wobei die Verarbeitungseinheit (130) konfiguriert ist, um:

eine Vielzahl von Magnitude-Werten entsprechend den zweiten Drei-Achsen-Beschleunigungswerten zu berechnen;
Berechnen eines Mittelwertes der Magnitude-Werte; und
Bestimmen, ob der Durchschnittswert höher als ein erster vorbestimmter Schwellenwert ist;
falls ja, Bestimmen, dass der Benutzer im gehenden Zustand ist; und
falls nicht, Bestimmen, dass der Benutzer nicht im gehenden Zustand ist.

8. Die elektronische Vorrichtung (100) nach Anspruch 6 oder 7, wobei die Verarbeitungseinheit (130) konfiguriert ist, um:

Filtern (S610) eines Rauschens in den Referenzwerte;
Berechnen (S620) einer Vielzahl von Steigungswerten entsprechend der Referenzwerte;
Akkumulieren (S630) eines Zählwertes wenn Plus- und Minuszeichen von zwei kontinuierlichen Steigungswerte aus den Steigungswerten unterschiedlich sind und ein Unterschied zwischen den zwei kontinuierlichen Steigungswerten größer als ein zweiter vorbestimmter Schwellenwert ist; und
Teilen (S640) der Zählwerte durch 2, um die Anzahl der Schritte entsprechend den zweiten Drei-Achsen-Beschleunigungswerten zu erhalten.

9. Die elektronische Vorrichtung (100) nach Anspruch 8, wobei die Verarbeitungseinheit (130) konfiguriert ist zum:

Bestimmen (S650), ob der Durchschnitt zwischen einem ersten Schätzwert und einen zweiten Schätzwert liegt;
falls ja, Multiplizieren (S660) der Anzahl von Schritten mit einen bestimmten Parameter, um die Anzahl der Schritte zu aktualisieren; und
falls nicht, beibehalten (S670) der Anzahl von Schritten.

10. Die elektronische Vorrichtung (100) nach Anspruch 9, wobei der spezifische Parameter ist:

$$\frac{\alpha_{motion} - \alpha_{walk}}{\alpha_{motion} - \alpha_{walk} + \varepsilon}$$

wobei $a_{walk}$ der erste Schätzwert ist, $a_{motion}$ ist der zweite Schätzwert $\varepsilon$, ist ein Fehterrate-Schätzwert.

**Revendications**

1. Une méthode de comptage de pas, adaptée à un appareil électronique (100), la méthode de comptage de pas comprenant :

   l'obtention (S210) d'une orientation et d'une pluralité de premières valeurs d'accélération suivant trois axes de l'appareil électronique (100) ;
   la suppression (S220) d'un rapport spécifique d'une accélération gravitationnelle de chacune des premières valeurs d'accélération suivant trois axes selon l'orientation pour générer une pluralité de secondes valeurs d'accélération suivant trois axes;
   le calcul (S230) d'une pluralité de valeurs de produits intrinsèques et d'une pluralité de valeurs de produits extrinsèques selon les secondes valeurs d'accélération suivant trois axes ;
   la détermination (S240) du fait de savoir si un utilisateur de l'appareil électronique (100) est dans un mode de marche selon les secondes valeurs d'accélération suivant trois axes ;
   dans l'affirmative, la fixation (S250) des valeurs de produits intrinsèques en tant que valeurs de référence ;
   dans la négative, la fixation (S260) des valeurs de produits extrinsèques en tant que valeurs de référence ; et
   le calcul (S270) d'un nombre de pas correspondant aux secondes valeurs d'accélération suivant trois axes en fonction des valeurs de références.

2. La méthode de comptage de pas telle que revendiquée dans la revendication 1, dans laquelle l'étape de détermination du fait de savoir si l'utilisateur de l'appareil électronique (100) est dans un mode de marche en fonction des secondes valeurs d'accélération suivant trois axes comporte :

   la calcul d'une pluralité d'amplitudes correspondant aux secondes valeurs d'accélération suivant trois axes ;
   le calcul d'une moyenne des valeurs d'amplitudes ; et
   la détermination de savoir si la moyenne est supérieure à un premier seuil ;
   dans l'affirmative, la détermination du fait de savoir si l'utilisateur est dans le mode de marche ; et
   dans la négative la détermination du fait de savoir si l'utilisateur n'est pas dans le mode de marche.

3. La méthode de comptage de pas telle que revendiquée dans la revendication 1 ou 2, dans laquelle l'étape de calcul du nombre de pas correspondant aux secondes valeurs d'accélération suivant trois axes en fonction des valeurs de références comporte :

   le filtrage (S610) d'un bruit au sein des valeurs de références ;
   le calcul (S620) d'une pluralité de valeurs de pentes correspondant aux valeurs de référence ;
   l'accumulation (S630) d'une valeur de comptage lorsque sont distinctes les signes positifs et négatifs des valeurs de pentes continues au sein des valeurs de pentes et lorsqu'une différence entre les deux valeurs de pentes continues est supérieure à un second seuil prédéterminé ; et
   la division (S640) de la valeur de comptage par 2 afin d'obtenir un nombre de pas correspondant aux secondes valeurs d'accélération suivant les trois axes.

4. La méthode de comptage de pas telle que revendiquée dans l'une quelconque des revendications 1 à 3, dans laquelle à la suite de l'étape de division par 2 de la valeur de comptage dans le but d'obtenir le nombre de pas correspondant aux secondes valeurs d'accélération suivant trois axes, la méthode comporte en outre :

   la détermination (S650) du fait de savoir si la moyenne se situe entre une première valeur estimée et une seconde valeur estimée ;

      dans l'affirmative, la multiplication (S660) du nombre de pas par un paramètre spécifique pour mettre à jour le nombre de pas ; et
      dans la négative, le maintien inchangé (S670) du nombre de pas.

5. La méthode de comptage de pas telle que revendiquée dans l'une quelconque des revendications 1 à 4, dans laquelle le paramètre spécifique est :

$$\frac{a_{motion} - a_{walk}}{a_{motion} - a_{walk} + \varepsilon}$$

dans lequel $a_{walk}$ est la première valeur estimée, $a_{motion}$ est la seconde valeur estimée, $\varepsilon$ est une valeur estimée de taux d'erreur.

6. Un appareil électronique (100), comprenant :

une unité de détection (110), détectant une orientation et une pluralité de premières valeurs d'accélération suivant trois axes de l'appareil électronique (100) ;
une unité de stockage (120), pour le stockage d'une pluralité de modules ; et
une unité de traitement (130), couplée à l'unité de détection (110) et à l'unité de stockage, et accédant aux modules en vue de l'exécution des étapes suivantes ;

l'obtention (S210) d'une orientation et d'une pluralité de premières valeurs d'accélération suivant trois axes de l'appareil électronique (100) ;
la suppression (S220) d'un rapport spécifique d'une accélération gravitationnelle de chacune des premières valeurs d'accélération suivant trois axes selon l'orientation dans le but de générer une pluralité de secondes valeurs d'accélération suivant trois axes;
le calcul (S230) d'une pluralité de valeurs de produits intrinsèques et d'une pluralité de valeurs de produits extrinsèques selon les secondes valeurs d'accélération suivant trois axes ;
la détermination (S240) du fait de savoir si un utilisateur de l'appareil électronique (100) est dans un mode de marche selon les secondes valeurs d'accélération suivant trois axes ;
dans l'affirmative, la fixation (S250) des valeurs de produits intrinsèques en tant que valeurs de référence ;
dans la négative, la fixation (S260) des valeurs de produits extrinsèques en tant que valeurs de référence ; et
le calcul (S270) d'un nombre de pas correspondant aux secondes valeurs d'accélération suivant trois axes en fonction des valeurs de références.

7. L'appareil électronique (100) tel que revendiqué dans la revendication 6, dans lequel l'unité de traitement (130) est configuré pour :

calculer une pluralité d'amplitudes correspondant aux secondes valeurs d'accélération suivant trois axes ;
calculer une moyenne des valeurs d'amplitudes ; et
déterminer si la moyenne est supérieure à un premier seuil ;

dans l'affirmative, déterminer si l'utilisateur est dans le mode de marche ; et
dans la négative, déterminer si l'utilisateur n'est pas dans le mode de marche.

8. L'appareil électronique (100) tel que revendiqué dans la revendication 6 ou 7, dans lequel l'unité de traitement (130) est configuré pour :

filtrer (S610) un bruit au sein des valeurs de références ;
calculer (S620) une pluralité de valeurs de pentes correspondant aux valeurs de référence ;
accumuler (S630) une valeur de comptage lorsque sont distinctes les signes positifs et négatifs des valeurs de pentes continues au sein des valeurs de pentes et lorsqu'une différence entre les deux valeurs de pentes continues est supérieure à un second seuil prédéterminé ; et
diviser (S640) la valeur de comptage par 2 afin d'obtenir un nombre de pas correspondant aux secondes valeurs d'accélération suivant les trois axes.

9. L'appareil électronique (100) tel que revendiqué dans la revendication 8, dans lequel l'unité de traitement (130) est configurée pour :

déterminer (S650) si la moyenne se situe entre une première valeur estimée et une seconde valeur estimée ;

dans l'affirmative, multiplier (S660) le nombre de pas par un paramètre spécifique pour mettre à jour le nombre de pas ; et

dans la négative, maintenir inchangé (S670) le nombre de pas.

**10.** L'appareil électronique (100) tel que revendiqué dans la revendication 9, dans lequel le paramètre spécifique est :

$$\frac{a_{motion} - a_{walk}}{a_{motion} - a_{walk} + \varepsilon}$$

dans lequel $a_{walk}$ est la première valeur estimée, $a_{motion}$ est la seconde valeur estimée, $\varepsilon$ est une valeur estimée de taux d'erreur.

110                                    120

Detection unit                    Storage unit

                    130

            Processing unit                    100

                        Electronic apparatus

# FIG. 1

Obtain an orientation and a plurality of first three-axis acceleration values of the electronic apparatus — S210

Remove a specific ratio of an acceleration of gravity from each of the first three-axis acceleration values according to the orientation to generate a plurality of second three-axis acceleration values — S220

Calculate a plurality of inner product values and a plurality of outer product values according to the second three-axis acceleration values — S230

Determine whether a user of the electronic apparatus is in a walking status according to the second three-axis acceleration values — S240

Yes          No

S250          S260

Set the inner product values as reference values

Set the outer product values as the reference values

S270

Calculate a number of steps corresponding to the second three-axis acceleration values according to the reference values

# FIG. 2

FIG. 3A

FIG. 3B

FIG. 3C

FIG. 4

FIG. 5

Filter a noise in the reference values ⌐~S610

Calculate a plurality of slope values corresponding to the reference values ⌐~S620

Accumulate a counting value when plus and minus signs of two continuous slope values in the slope values are different and a difference between the two continuous slope values is greater than a second predetermined threshold value ⌐~S630

Divide the counting value by 2 to obtain the number of steps corresponding to the second three-axis acceleration values ⌐~S640

Determine whether the average is between a first estimation value and a second estimation value ⌐S650

Yes

No

S660⌐ Multiply the number of steps by a specific parameter to update the number of steps

S670 Maintain the number of steps

S270

FIG. 6

FIG. 7

FIG. 8

FIG. 9

1010

Outer product
operation

1020

Moving
average

1030

Obtain the
slope value

1040

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1994883 A1